# EUROPEAN PATENT SPECIFICATION

(11) **EP 0 707 854 B1**
(45) Date of publication and mention of the grant of the patent: **22.02.2006**
(21) Application number: 95112838.8
(22) Date of filing: 10.06.1986
(51) Int. Cl.: A61K 33/24, A61K 31/60, A61K 31/535, A61K 31/43, A61K 31/415, A61K 31/29

(54) **Compositions for the treatment of gastronitestinal disorders containing bismuth and an antimicrobial**
Pharmazeutische Zusammensetzung zur Behandlung gastrointestinaler Beschwerden, die Wismut und ein antimikrobielles Mittel enthält
Compositions pour le traitement des maladies gastrointestinales, contenant du bismuth et un agent antimicrobien

(30) Priority: 13.06.1985 US 744841
(43) Date of publication of application: 24.04.1996
(62) Divisional of application: 91114034.1
(73) Proprietor: Marshall, Barry James, Dr., Virginia 22936 (US)
(72) Inventor: Marshall, Barry James, Dr., Virginia 22936 (US)
(74) Representative: Samuels, Lucy Alice

(56) References cited:
- FR-M- 6 531
- US-A- 4 016 268
- US-A- 4 118 480
- UNLISTED DRUGS, vol. 22, no. 11, November 1970, NEW YORK, US, page 163b XP002028541 "Aldefur"

## Description

### BACKGROUND OF THE INVENTION

This invention relates to the treatment of gastrointestinal disorders in humans and other animals.

Factors adversely affecting the function of the gastrointestinal system in humans are exceedingly varied in their nature. Such disorders may arise in the upper or lower gastrointestinal tracts or both. There is a broad range of causes of gastrointestinal disorders, including genetic, physiological, environmental, and psychogenic factors. Accordingly, the diagnosis and management of these disorders can be exceptionally difficult. A detailed discussion of gastrointestinal tract functions, disorders, causes, and treatments can be found in Spiro, Clinical Gastroenterology (3d. edition 1983).

Among the chronic disorders of the upper gastrointestinal tract are those which fall under the general categories of gastritis and peptic ulcer disease. (The upper gastrointestinal tract is generally defined as including the esophagus, the stomach, the duodenum, the jejunum, and Ilium.) Gastritis is, by definition, typified by an inflammation of the stomach mucosa. In practice though, the disorder is manifested by a broad range of poorly-defined, and heretofore inadequately treated, symptoms such as indigestion, "heart burn", dyspepsia and excessive eructation. A general discussion of gastritis appears in B. J. Marshall and J. R. Warren, "Unidentified Curved Bacilli in the Stomach of Patients with Gastritis and Peptic Ulceration". The Lancet, 1311-1315 (1984)" and in R. Greenlaw, et al.. "Castroduodenitis, A Broader Concept of Peptic Ulcer Disease". 25 Digestive Diseases and Sciences 660-672 (1980).

Peptic ulcers are lesions of the gastrointestinal tract lining, characterized by loss of tissue due to the action of digestive acids and pepsin. It has been generally held that peptic ulcers are caused either by gastric hypersecretion, or [more often] by decreased resistance of the gastric lining to digestive acids and pepsin. The medical literature is replete with methods for treating ulcers, including modification of the diet, surgical removal of the lesions, and the use of drugs. Such drugs include: antacids, which serve to counteract excess gastric secretions; anticholinergics, which reduce acid secretion; H₂ antagonists, which also block the release of gastric acids; Prostaglandins, which increase the resistance of the gastric lining to digestive fluids, and may also inhibit acid secretion; prokinetic agents, which enhance gastrointestinal tract motility; and compositions which form protective barriers over gastric lesions. Prescription and non-prescription drug therapies are generally described in Carnet, "Antacid Products", Handbook of Non-prescription Drugs, Chapter 3 (7th edition, 1982). One group of drugs which are thought to be effective due to coating of ulcer sites and forming protective barriers is the bismuth-containing drugs. See, for example, Kao, et al., "Selective Coating of Gastric Ulcers by Tripotassium Dicitrato Bismuthate in the Rat", 82 Gastroenterology 864-870 (1982).

Regardless of the particular drug composition used in treating gastrointestinal disorders, such as peptic ulcer disease, the treatment is often imprecise and incomplete. Actual "cures", i.e., successful treatment resulting in total remission of disease, are very often not effected. See, A. J. McLean, et al., "Cytoprotective Agents and Ulcer Relapse", 142 The medical Journal of Australia, Special Supplement S25-S28 (1985). Furthermore, many conventional treatments may render subjects hypochlorhydric (i.e., with low levels of hydrochloric acid in the stomach) which may predispose them to other disorders, e.g., gastrointestinal infections, halitosis, and gastric carcinomas.

It has now been discovered that certain methods of treatment, involving the administration of bismuth in defined forms and the administration of the antimicrobial metronidazole are effective for the treatment of gastrointestinal disorders. In particular, as compared to treatment regimens known in the art, these methods cure, or afford lower relapse rates of, gastritis and peptic ulcer disease. These methods also afford other benefits in the treatment and management of subjects having gastrointestinal diseases, such as in not rendering treated subjects hypochlorhydric.

### SUMMARY OF THE INVENTION

The present composition provides uses, defined in the independent claims, of bismuth and of antimicrobial metronidazole, in the manufacture of a medicament for the treatment of a human or lower animal subject having a gastrointestinal disorder, in a method comprising administering, to said subject from about 50 milligrams to about 5000 milligrams of bismuth, per day, for from 3 to 56 days, and administering to said subject a safe and effective amount of an antimicrobial comprising metronidazole, per day, for from 1 to 21 days.

The bismuth is selected from the group consisting of bismuth aluminate, bismuth subcarbonate, bismuth citrate, bismuth subgalate, bismuth subnitritate, bismuth cartrate, bismuth subsalicylate and mixtures thereof.

Typically, the antimicrobial is administered at a level of from about 100 milligrams to about 10.000 milligrams, per day. Preferably the bismuth is administered for from 1 to 21 days prior to initial administration of the antibiotic. Preferred methods comprise administration of bismuth and administration of antimicrobials to human or lower animal subjects that have been tested for the presence of infection by pyloric campylobacter or other pathogenic organisms in the upper gastrointestinal tract, with positive results. A preferred test for such infection is through the detection of urease enzyme in the stomach.

This invention can make use of compositions for the treatment of gastrointestinal disorders, comprising a safe and effective amount of bismuth and a safe and effective amount of metronidazole. These compositions are particularly useful in the present invention.

### DESCRIPTION OF INVENTION

The methods comprise treatment of humans or lower animals, having gastrointestinal disease, by administering bismuth and metronidazole. Specific compounds and compositions to be used in the processes and compositions of the present invention must, accordingly, be pharmaceutically-acceptable. As used herein, such a "pharmaceutically-acceptable" component is one which is suitable for use with humans and/or animals without undue adverse side effects (such as toxicity irritation, and allergic response) commensurate with a reasonable benefit/risk ratio. Further, as used herein, the term "safe and effective amount" refers to the quantity of a component which is sufficient to yield a desired therapeutic response without undue adverse effects (such as toxicity, irritation, or allergic response) commensurate with a reasonable benefit/risk ratio when used in the manner of this invention. The specific "safe and effective amount" will, obviously, vary with such factors as the particular condition that is being treated, the severity of the condition, the duration of the treatment, the physical condition of the patient, the nature of concurrent therapy (if any), and the specific formulations employed.

Specifically, the processes for the treatment of a human or lower animal subject having a gastrointestinal disorder, comprise administering to said subject from about 50 milligrams to about 5000 milligrams of bismuth, per day, for from 3 to 56 days, and administering to said subject a safe and effective amount of an antimicrobial comprising metronidazole, per day, for from 1 to 21 days.

As used herein, "gastrointestinal disorder" encompasses any disease or other disorder of the upper gastrointestinal tract of a human or lower animal. Such gastrointestinal disorders include, for example: disorders not manifested by presence of ulcerations in the gastric mucosa (herein "non-ulcerative gastrointestinal disorder"), including chronic or atrophic gastritis, non-ulcer dyspepsia, esophogeal reflux disease and gastric motility disorders; and "peptic ulcer disease", i.e., gastric, duodenal and jejunal ulcers. In particular, "gastrointestinal disorder" refers to such disorders of the upper gastrointestinal tract caused or mediated by bacteria, including campylobacter-like organisms (herein "CLO"), e.g., Campylobacter pyloridis. Such CLO include those described in J. R. Warren and B. J. Marshall, "Unidentified Curved Bacilli on Gastric Epithelium in Active Chronic Gastritis", The Lancet 1273-1275 (1983), incorporated by reference herein, and G. Kasper and N. Dickgiesser, "Isolation from Gastric Epithelium of Campylobacter-like Bacteria that are Distinct from 'Campylobacter Pyloridis''', The Lancet 111-112 (1985).

The processes encompass processes wherein the administering of bismuth and the adminstering of metronidazole are performed simultaneously (beginning and ending on the same day), concurrently (overlapping), or consecutively (sequential, but wherein the course of treatment is substantially continuous). Preferably, though, the step of administering the metronidazole is not commenced prior to commencing the step of administering the bismuth.

As used herein, "administering" refers to any method which, in sound medical practice, delivers the compounds or compositions used in this invention to the subject to be treated in such a manner so as to be effective in the treatment of the gastrointestinal disorder. Preferably, then, the bismuth is administered orally. Also preferably, the antimicrobials are administered either orally, intraveneously, or any other method which effects systemic distribution, or local distribution to the site of the CLO infection, of the antibiotic in the subject. Oral ingestion of the antibiotic is a preferred method of administering the antibiotic in the processes of this invention.

### Bismuth:

The processes involve administration of from about 50 milligrams to about 5000 milligrams of bismuth, per day, for from 3 to 56 days. (As used herein, the quantity of bismuth is by weight of elemental bismuth. Thus, the actual weight of a bismuth-containing compound will be greater.) Preferably, from about 500 milligrams to about 1500 milligrams of bismuth are administered, per day. The preferred duration of bismuth administration will vary according to the specific gastrointestinal disorder to be treated. In general, though, in methods for treatment of non-ulcerative gastrointestinal disorders, the bismuth is administered for from 3 to 21 days. The bismuth is preferably adminstered, in methods for treatment of peptic ulcer disease, for from 14 to 56 days.

In the processes, the bismuth is preferably administered as a pharmaceutically-acceptable salt. Such bismuth salts include, for example, bismuth aluminate, bismuth subcarbonate, bismuth citrate, bismuth subgalate, bismuth subnitrate, bismuth tartrate, bismuth subsalicylate, and mixtures thereof. Bismuth citrate, bismuth tartrate, bismuth subsalicylate, and mixtures thereof are preferred bismuth salts for use in this invention. The bismuth useful herein may be administered alone, or in combination with other pharmaceutically-acceptable components, in a bismuth-containing composition. A variety of such compositions containing bismuth salts are commercially-available, including for example, Noralac, containing bismuth aluminate, alginic acid, and magnesium carbonate (manufactured by North American Pharmaceuticals), Roter bismuth, containing bismuth subnitrate (sold by Roter Laboratories), Fensobar Polvo, containing bismuth subcarbonate among other Materials (manufactured by USV Pharmaceutical Corporation), and Pepto-Bismol, containing bismuth subsalicylate (sold by The Procter 6 Camble Company).

### Antimicrobial:

The processes of tne present invention also include administration of a safe and effective amount of an antimicrobial, which comprises metronidazole per day, for from 1 to 21 days. Typically, the antimicrobial is administered at a level of from about 100 milligrams to about 10.000 milligrams, per day. Preferably, the antimicrobial is administered for from 1 day to 14 days. The specific dosage of antimicrobial to be administered, as well as the duration of antimicrobial treatment, are mutually dependent, and will also depend upon such factors as the specific antimicrobial used, the resistance pattern of the infecting organism to the antimicrobial used, the ability of the antimicrobial to reach minimum inhibitory concentrations at the site of the infection, the nature and extent of other infections (if any), the personal attributes of the subject, compliance with the treatment regimen, and the presence and severity of any side effects of the treatment.

A wide variety of antimicrobials are useful in this invention, although it is essential that the antimicrobial comprises metronidazole. As used herein, such "antimicrobials" refer to any naturally-occurring, synthetic or semi-synthetic compound or composition, or mixture thereof, which is safe for human use as used in the processes of this invention, and is effective in killing or substantially inhibiting the growth of CLO when used in the processes of this invention. Antibiotics are among the preferred antimicrobials useful herein. Such antibiotics can be generally classified by chemical composition, into the following principal groups: the aminoglycosides, such as gentamicin, neomycin, kanamycin, and streptomycin; the macrolides, such as erythromycin, clindamycin, and rifampin; the penicillins, such as penicillin G, penicillin V, ampicillin and amoxycillin; the polypeptides, such as bacitracin and polymyxin; the tetracyclines, such as tetracycline, chlortetracycline, oxytetracycline, and doxycycline; the cephalosporins, such as cephalexin and cephalothin; and such miscellaneous antibiotics as chloramphenicol and clindamycin. These antibiotics can be generally said to function in one of four ways: inhibition of cell walls synthesis, alteration of cell wall permeability, inhibition of protein synthesis, or inhibition of nucleic acid synthesis.

Other antimicrobials useful herein include the sulfonamides; nitrofurans, such as nitrofurazone nitrofurantoin, and furozolidone; and tinidazole, and nimorazole. Antimicrobials among those useful herein are described in the following publications, incorporated by reference herein: Remington's Pharmaceutical Sciences (15th edition 1975); F. H. Meyers, et al., Review of Medical Pharmacology (7th edition 1980); Gaddum's Pharmocology (8th edition 1978); and A. Goodman, A. G. Goodman and L. S. Gilman, The Pharmacological Basis of Therapeutics (6th edition 1980).

While any of these antimicrobials may be used, penicillin, erythromycin, doxycycline, tinidazole, amoxycillin, ampicillin, and nitrofurantoin are among the preferred antimicrobials for use in the present invention and metronidazole is essential. In a preferred method of treatment, a sample of CLO is obtained from the stomach of the subject to be treated, as by biopsy, aspiration, or by other suitable method, and the organism cultured and tested for sensitivity to the various antimicrobials useful herein. Preferably such sensitivity testing is by determination of the relative minimum inhibitory concentrations of the antimicrobials using broth or plate dilution techniques. The antimicrobial found to be most effective against the cultured bacteria (i.e., effective at the lowest minimum inhibitory concentration) is then selected for use in the methods of this invention.

As stated above, the specific preferred quantity of antimicrobial, and duration of treatment used in the methods of this invention will, in addition to other factors, depend upon the particular antimicrobial used and its pharmacology. In general, though, the tetracyclines are preferably administered at a level of from about 100 milligrams to about 2000 milligrams, per day. Macrolides (such as erythromycin) are preferably administered at a level of from about 1000 milligrams to about 4000 milligrams, per day. Penicillins are preferably administered at a level of from about 500 milligrams to about 3000 milligrams, per day. The aminoglycosides (such as neomycin) are, preferably, administered at a level of from about 100 milligrams to about 8000 milligrams, per day. Nitrofurans (such as nitrofurantoin) are administered preferably at levels of from about 100 milligrams to about 800 milligrams, per day. Preferably, metronidazole is administered at a level of from about 500 to about 2000 milligrams, per day.

The specific method of administering the antimicrobial, according to the processes of this invention, may depend upon such factors as the particular antimicrobial used, the site of infection, the amount of antimicrobial to be administered per day, the presence of any adverse side effects, and the interactions (if any) between the antimicrobial and the bismuth. Thus, the antimicrobials may be administered under the process of this invention by single daily doses, or by administration in two, three, four, or more doses per day. One factor, in particular, is potential interaction between the antimicrobial and the bismuth administered under these processes. For example, the presence of bismuth is known to adversely affect the efficacy of the tetracyclines. See, for example, C.D. Ericsson, et. al., "Influence of Subsalicylate Bismuth on Absorption of Doxycycline" 247 J. of American Medical Assoc. 2266 (1982). Hence, it is preferred to adminster those antimicrobials that are subject to adverse bismuth interaction by methods that minimize such interactions, i.e., by minimizing the simultaneous presence of antimicrobial and bismuth in the stomach. Such methods include one or more of the following: staggered oral dosing of the bismuth and antimicrobial, through discrete administration of each compound or composition separated by at least (preferably) two hours between dosages; oral administration of the antimicrobial in an enterically coated form, i.e., coating of the antimicrobial which prevents dissolution of the antimicrobial in the stomach; use of optional processes of this invention, wherein the step of administering bismuth is terminated prior to commencing the step of orally administering the antimicrobial; and administering the antimicrobial by a non-oral route, e.g., by intraveneous or intramuscular injection.

### Bismuth/Antimicrobial Compositions:

The present invention can also provide compositions, for the treatment of gastrointestinal disorders, comprising a safe and effective amount of bismuth and a safe and effective amount of an antimicrobial comprising metronidazole. Typically, these compositions comprise:
(a) from about 50 milligrams to about 5000 milligrams of bismuth; and
(b) from about 100 milligrams to about 10,000 milligrams of antimicrobial comprising metronidazole.

Preferably, the bismuth salt is present at a level of from about 250 milligrams to about 1000 milligrams. Also preferably, the antimicrobial is present at a level of from about 100 milligrams to about 1000 milligrams.

The compositions may contain optional components which affect the physical and therapeutic characteristics of the present compositions. In particular, a variety of pharmaceutically-acceptable carriers and excipients may be included, depending upon the particular dosage form to be used. Various oral dosage forms can be used, including such solid forms as tablets, capsules, granules and bulk powders. Tablets can be compressed, tablet triturates, enteric-coated, sugar-coated, film-coated or multiple compressed, containing suitable binders, lubricants, diluents, disintegrating agents, coloring agents, flavoring agents, flow-inducing agents and melting agents. Liquid oral dosage forms include aqueous solutions, emulsions, suspensions, solutions and/or suspensions reconstituted from non-effervescent granules and effervescent preparations reconstituted from effervescent granules, containing suitable solvents, preservatives, emulsifying agents, suspending agents, diluents, sweeteners, melting agents, coloring, and flavoring agents.

Specific examples of pharmaceutically-acceptable carriers and excipients that may be used to formulate oral dosage forms of the present invention are described in U.S. Patent 3,903,297, Robert, issued September 2, 1975, incorporated by reference herein. Techniques and compositions for making dosage forms useful herein are described in the following references, all incorporated by reference herein: 7 Modern Pharmaceutics, Chapters 9 and 10 (Banker and Rhodes, editors, 1979); an Lieberman, et al., Pharmaceutical Dosage Forms: Tablets (1981); and Ansel, Introduction to Pharmaceutical Dosage Forms (2d edition, 1976).

As discussed above, care must be taken in avoiding any interactions between the bismuth compound or composition and the particular antimicrobial used in these compositions. Accordingly, in preferred compositions of this invention, the antimicrobial is physically separated from the bismuth, in such a manner so that the antimicrobial and the bismuth are not simultaneously dissolved in the stomach. Hence, a preferred composition of this invention comprises a capsule containing bismuth particles and enterically-coated antimicrobial particles.

The compositions of this invention may be used according to the methods of this invention, by administering the composition from 1 to 7 times, per day, for from 3 to 21 days. The specific frequency of administration will depend upon such factors as the specific bismuth compound or composition and antimicrobial used, the levels at which the components are incorporated in the composition, the nature and severity of the condition to be treated, and the nature of any concurrent therapy (if any).

### Optional Components and Methods:

The methods incorporate optional steps modifying the methods of treatment. Such optional steps may also utilize optional components or compositions. Such optional components or compositions must not, however, adversely affect the therapeutic activity of the bismuth or of the antimicrobial used in the present methods.

A preferred optional method of involves a delay, preferably of from 1 to 21 days, more preferably from 1 to 10 days, more preferably from 1 to 5 days, in the administration of antimicrobial after initial administration of a bismuth salt. Such preferred methods for the treatment of a human or lower animal subject having a gastrointestinal disorder thus comprise the steps of:
a) administering to said subject from about 50 to about 5000 milligrams of a bismuth salt, per day, for from 3 to 56 days; and
b) administering to said subject a safe and effective amount of antimicrobial comprising metronidazole per day, for from about 1 to about 21 days;

wherein said step of administering antimicrobial is commenced from 1 to 21 days after the commencement of said step of administering bismuth. Also preferably, a sample of gastric material is obtained from the stomach of the subject, and the infecting CLO cultured and tested for sensitivity to the antimicrobials useful herein (as described above) during the period of initial administration of bismuth but prior to administration of antimicrobial.

Another preferred method includes a step for the detection of CLO in the upper gastrointestinal tract of the human or lower animal subject. The methods for detection useful in such preferred steps include gram stains of gastric tissues (obtained, for example, by biopsyl, serologic tests to detect the presence of antibodies to the organisms, tests of body fluids to detect the presence of metabolites of the organisms, silver or other specially stained tissue sections of tissues, and detection of urease enzyme in the stomach of the subject. Such detection steps are also described in copending application 86304408.7 (EP-A-206626) Marshall, "Methods for the Treatment of Gastrointestinal Disorders".

One preferred method of detection, useful in a preferred process, is the detection of urease enzyme (urea amidohydrolasel in the stomach of the human or lower animal having a gastrointestinal disorder. Such a detection step may include, for example, obtaining a sample of gastric fluid (e.g., from a gastric tube or vomitus) or of gastric mucosa (e.g., by biopsyl and analyzing the material for the presence of urease enzyme. Such methods involve obtaining a sample of gastric mucosa and placing said sample into a composition which comprises:
a) urea, at a concentration of from about 10 to about 40 grams per liter;
b) a bactericide, at a concentration of from about 1 to about 5 grams per liter;
c) an indicator having a pKₐ of from about 6.5 to about 8.5, at an effective concentration; and
d) water;

wherein said composition has a pH of from about 5.0 to about 6.5 and said pH is at least one pH unit lower than the pKₐ of said indicator. Preferably, the composition contains a gelling agent, such as a non-nutritive agar, at a concentration of from about 5 to about 50 grams per liter. Typically the indicator is present at a concentration of from about 20 to about 100 milligrams per liter. (As used herein, all concentrations are by weight of component per total volume of composition.) A change in the color of the composition indicates the presence of urease enzyme, and the presence of a gastrointestinal disorder.

The diagnostic step is preferably performed prior to the step of administering bismuth. Also preferably, the diagnostic step is repeated during the step of administering bismuth, and the step of administering bismuth is terminated after the diagnostic step yields a negative result. Thus, a preferred method, for the treatment of a human or lower animal subject having a gastrointestinal disorder, comprises the steps of:
a) performing a diagnostic test on said subject for the detection of a CLO infection of said subject; and, upon said diagnostic test,
b) administering to said Subject from about 50 milligrams to about 5000 milligrams of bismuth as defined above, per day, for a period of time ending when said subject is tested with said diagnostic test and a negative result is obtained; followed by
c) administering from about 100 milligrams to about 10000 milligrams of an antimicrobial comprising metronidazole, per day, for from 1 to 23 days.

The following non-limiting example illustrates the present invention.

### EXAMPLE I

A human subject, suffering from peptic ulcer disease, is treated by a method of this invention. Specifically, a sample of vomitus is obtained from the individual and analyzed for the presence of urease. After detecting urease, the individual is then treated by administering 500 milligrams of bismuth subsalicylate, per day (two doses per day) for 28 days. After the fifth day (commencing on the sixth day) of bismuth treatment, the subject is also treated by administering 750 milligrams of metronidazole, per day, for 14 days. (Hence, the bismuth treatment continues for 9 days after the last day of antimicrobial treatment.) The subject is then endoscoped, revealing healing of the peptic ulcer lesion.

## Claims

1. The use of bismuth for the manufacture of a medicament for the treatment of a human or lower animal subject having an infectious gastrointestinal disorder, said treatment comprising administering to said subject from 50 milligrams to 5,000 milligrams of bismuth, per day, for from 3 to 56 days, and administering to said subject a safe and effective amount of an antimicrobial comprising metronidazole, per day, for from 1 to 21 days, wherein the bismuth is selected from the group consisting of bismuth aluminate, bismuth subcarbonate, bismuth citrate, bismuth subgalate, bismuth subnitrate, bismuth tartrate, bismuth subsalicylate, and mixtures thereof.

2. The use of bismuth according to any preceding claim, wherein from 500 to 1,500 milligrams of bismuth are administered to said subject per day.

3. The use of bismuth according to any preceding claim, wherein said infectious gastrointestinal disorder is a non-ulcerative gastrointestinal disorder.

4. The use of bismuth according to claim 3, wherein said bismuth is administered for from 3 to 21 days.

5. The use of bismuth according to claim 1 or claim 2, wherein said infectious gastrointestinal disorder is a peptic ulcer disease.

6. The use of bismuth according to claim 5, wherein said bismuth is administered for from 14 to 56 days.

7. The use of bismuth according to any one of claims 1 through 6, wherein said bismuth is bismuth subsalicylate.

8. The use of metronidazole for the manufacture of a medicament for the treatment of a human or lower animal having an infectious gastrointestinal disorder, said treatment comprising administering to said subject from 100 milligrams to 10,000 milligrams per day of an antimicrobial comprising metronidazole, and administering to said subject from 50 milligrams to 5,000 milligrams of bismuth per day, for from 3 to 56 days, wherein the bismuth is selected from the group consisting of bismuth aluminate, bismuth subcarbonate, bismuth citrate, bismuth subgalate, bismuth subnitrate, bismuth tartrate, bismuth subsalicylate, and mixtures thereof.

9. The use of metronidazole according to claim 8, wherein said antimicrobial is administered for from 1 to 14 days.

10. The use of metronidazole according to claim 8 or claim 9, wherein said administering of said bismuth and said administering of said antimicrobial are performed in steps, whereby the step of administering said antimicrobial is commenced from 1 to 21 days after the commencement of the step of administering said bismuth.

11. The use of metronidazole according to claim 8 or claim 9, wherein said administering of said bismuth and said administering of said antimicrobial are performed in steps, whereby the step of administering said antimicrobial is commenced after the completion of the step of administering said bismuth.

## Revendications

1. Utilisation de bismuth pour la fabrication d'un médicament destiné au traitement d'un sujet humain ou animal inférieur souffrant d'un trouble gastro-intestinal infectieux, ledit traitement comprenant l'administration audit sujet de 50 milligrammes à 5000 milligrammes de bismuth par jour, pendant 3 à 56 jours, et l'administration audit sujet d'une quantité sans danger et efficace d'un antimicrobien comprenant du métronidazole, par jour, pendant 1 à 21 jours, où le bismuth est choisi dans le groupe constitué par l'aluminate de bismuth, le sous-carbonate de bismuth, le citrate de bismuth, le sous-galate de bismuth, le sous-nitrate de bismuth, le tartrate de bismuth, le sous-salicylate de bismuth, et leurs mélanges.

2. Utilisation de bismuth selon l'une quelconque des revendications précédentes, où 500 à 1500 milligrammes de bismuth sont administrés audit sujet par jour.

3. Utilisation de bismuth selon l'une quelconque des revendications précédentes, où ledit trouble gastro-intestinal infectieux est un trouble gastro-intestinal non ulcératif.

4. Utilisation de bismuth selon la revendication 3, où ledit bismuth est administré pendant 3 à 21 jours.

5. Utilisation de bismuth selon la revendication 1 ou la revendication 2, où ledit trouble gastro-intestinal infectieux est une maladie de type ulcère peptique.

6. Utilisation de bismuth selon la revendication 5, où ledit bismuth est administré pendant 14 à 56 jours.

7. Utilisation de bismuth selon l'une quelconque des revendications 1 à 6, où ledit bismuth est le sous-salicylate de bismuth.

8. Utilisation de métronidazole pour la fabrication d'un médicament destiné au traitement d'un humain ou d'un animal inférieur souffrant d'un trouble gastro-intestinal infectieux, ledit traitement comprenant l'administration audit sujet de 100 milligrammes à 10 000 milligrammes par jour d'un antimicrobien comprenant le métronidazole, et l'administration audit sujet de 50 milligrammes à 5000 milligrammes de bismuth par jour, pendant 3 à 56 jours, où le bismuth est choisi dans le groupe constitué par l'aluminate de bismuth, le sous-carbonate de bismuth, le citrate de bismuth, le sous-galate de bismuth, le sous-nitrate de bismuth, le tartrate de bismuth, le sous-salicylate de bismuth, et leurs mélanges.

9. Utilisation de métronidazole selon la revendication 8, ledit antimicrobien étant administré pendant 1 à 14 jours.

10. Utilisation de métronidazole selon la revendication 8 ou la revendication 9, où ladite administration dudit bismuth et ladite administration dudit antimicrobien s'effectuent en plusieurs étapes selon lesquelles l'étape d'administration dudit antimicrobien débute 1 à 21 jours après le commencement de l'étape d'administration dudit bismuth.

11. Utilisation de métronidazole selon la revendication 8 ou la revendication 9, où ladite administration dudit bismuth et ladite administration dudit antimicrobien s'effectuent en plusieurs étapes selon lesquelles l'étape d'administration dudit antimicrobien débute à la fin de l'étape d'administration dudit bismuth.

## Patentansprüche

1. Verwendung von Wismut für die Herstellung eines Arzneimittels zur Behandlung eines Menschen oder eines niederen Tieres mit einer infektiösen gastrointestinalen Erkrankung, wobei die Behandlung das Verabreichen an das Individuum von 50 mg bis 5.000 mg Wismut pro Tag für 3 bis 56 Tage und das Verabreichen an das Individuum einer sicheren und wirksamen Menge eines antimikrobiellen Mittels, umfassend Metronidazol, pro Tag für 1 bis 21 Tage umfaßt, wobei das Wismut gewählt ist aus der Gruppe, bestehend aus Wismutaluminat, Wismutsubcarbonat, Wismutcitrat, Wismutsubgalat, Wismutsubnitrat, Wismuttartrat, Wismutsubsalicylat und Mischungen hiervon.

2. Verwendung von Wismut nach dem vorhergehenden Anspruch, wobei 500 mg bis 1.500 mg Wismut pro Tag an das Individuum verabreicht werden.

3. Verwendung von Wismut nach irgendeinem der vorhergehenden Ansprüche, wobei die infektiöse gastrointestinale Erkrankung eine nicht-ulzeröse gastrointestinale Erkrankung ist.

4. Verwendung von Wismut nach Anspruch 3, wobei das Wismut für 3 bis 21 Tage verabreicht wird.

5. Verwendung von Wismut nach Anspruch 1 oder Anspruch 2, wobei die infektiöse gastrointestinale Erkrankung eine Ulkuskrankheit ist.

6. Verwendung von Wismut nach Anspruch 5, wobei das Wismut für 14 bis 56 Tage verabreicht wird.

7. Verwendung von Wismut nach irgendeinem der Ansprüche 1 bis 6, wobei das Wismut ein Wismutsubsalicylat ist.

8. Verwendung von Metronidazol für die Herstellung eines Arzneimittels zur Behandlung eines Menschen oder eines niederen Tieres mit einer infektiösen gaslrointestinalen Erkrankung, wobei die Behandlung das Verabreichen an das Individuum von 100 mg bis 10.000 mg pro Tag eines antimikrobiellen Mittels, umfassend Metronidazol, und das Verabreichen an das Individuum von 50 mg bis 5.000 mg Wismut pro Tag für 3 bis 56 Tage umfaßt, wobei das Wismut gewählt ist aus der Gruppe, bestehend aus Wismutaluminat Wismutsubcarbonat, Wismutcitrat, Wismutsubgatat, Wismutsubnitrat, Wismuttartrat, Wismutsubsalicylat und Mischungen hiervon.

9. Verwendung von Metronidazol nach Anspruch 8. wobei das antimikrobielle Mittel für 1 bis 14 Tage verabreicht wird.

10. Verwendung von Metronidazol nach Anspruch 8 oder Anspruch 9, wobei die Verabreichung des Wismuts und die Verabreichung des antimikrobiellen Mittels in Schritten durchgeführt werden, wobei der Schritt des Verabreichens des antimikrobiellen Mittels innerhalb von 1 bis 21 Tagen nach dem Beginn des Schrittes des Verabreichens von Wismut beginnt.

11. Verwendung von Metronidazol nach Anspruch 8 oder Anspruch 9, wobei die Verabreichung des Wismuts und die Verabreichung des antimikrobiellen Mittels in Schritten durchgeführt werden, wobei der Schritt des Verabreichens des antimikrobiellen Mittels nach der Beendigung des Schrittes des Verabreichens von Wismut beginnt.
